(19) Europäisches Patentamt
European Patent Office
Office européen des brevets

(11) Veröffentlichungsnummer : **0 151 282**
**B1**

# (12) EUROPÄISCHE PATENTSCHRIFT

(45) Veröffentlichungstag der Patentschrift : **07.09.88**

(51) Int. Cl.⁴ : **C 07 F 9/65**, C 07 F 15/00, B 01 J 31/24

(21) Anmeldenummer : **84115470.1**

(22) Anmeldetag : **14.12.84**

## (54) Optisch aktive 3,4-Bis(diphenylphosphino)-pyrrolidine, diese als chirale Liganden enthaltende Rhodiumkomplexe und deren Verwendung.

(30) Priorität : **31.01.84 DE 3403194**

(43) Veröffentlichungstag der Anmeldung : **14.08.85 Patentblatt 85/33**

(45) Bekanntmachung des Hinweises auf die Patenterteilung : **07.09.88 Patentblatt 88/36**

(84) Benannte Vertragsstaaten : **AT BE CH DE FR GB IT LI NL SE**

(56) Entgegenhaltungen :
**US-A- 4 343 741**
**CHEMISTRY LETTERS, Nr. 8, August 1983, Seiten 1203-1206, The Chemical Society of Japan; M. YATAGAI u.a.: "Effective asymmetric hydrogenation of dehydrodipeptides with rhodium(I) - New chiral diphosphinite systems"**
**ANGEW. CHEM. INT. ED. ENGL., Band 23, Nr. 6, 1984, Seiten 435-436, Verlag Chemie GmbH, Weinheim, DE; U. NAGEL: "Asymmetric hydrogenation of alpha-(Acetylamino)cinnamic acid with a novel rhodium compled; the design of an optimal ligand"**

(73) Patentinhaber : **Degussa Aktiengesellschaft**
**Weissfrauenstrasse 9**
**D-6000 Frankfurt am Main 1 (DE)**

(72) Erfinder : **Beck, Wolfgang, Prof. Dr.**
**Melanchthonstrasse 26**
**D-8000 München 83 (DE)**
Erfinder : **Nagel, Ulrich, Dr.**
**Freiherrnstrasse 12**
**D-8061 Weichs (DE)**

**EP 0 151 282 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue optisch aktive 3,4-Bis-(diphenylphosphino)-pyrrolidine, diese als chirale Liganden enthaltende Rhodiumkomplexe und deren Verwendung als Katalysatoren für die homogene asymmetrische Hydrierung unsubstituierter oder β-substituierter α-Acylamino-acrylsäuren.

Aus Chemistry Letters 1983, Seiten 1203 bis 1206, sind optisch aktive Pyrrolidin-Derivate der Formel

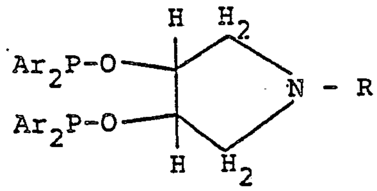

bekannt, in der Ar einen Phenylrest oder einen 4-Methoxyphenylrest und R einen Alkylrest oder einen ω-Dimethylaminoalkylrest bedeuten. Diese Pyrrolidin-Derivate können als chirale Liganden in Rhodiumkomplexen dienen, welche als Katalysatoren für die asymmetrische Hydrierung von Dehydrodipeptiden empfohlen werden. Bei der asymmetrischen Hydrierung von α-Acetamidozimtsäure ist jedoch die Stereoselektivität dieser bekannten Rhodiumkomplexe nur gering und es werden infolgedessen auch nur niedrige optische Ausbeuten erzielt.

Ein erster Gegenstand der Erfindung sind nun optisch aktive 3,4-Bis-(diphenylphosphino)-pyrrolidine der allgemeinen Formel

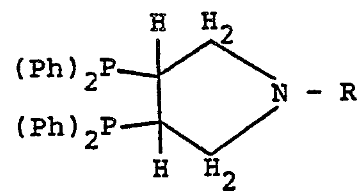

(I)

in der Ph einen Phenylrest und R Wasserstoff, einen Alkylrest, einen Arylalkylrest oder einen Acylrest bedeuten.

Die Grundsubstanz, nämlich das 3,4-Bis-(diphenylphosphino)-pyrrolidin selbst, kann in der Weise hergestellt werden, daß man optisch aktive Weinsäure mit Benzylamin zum 1-Benzyl-2,5-dioxo-3,4-dihydroxypyrrolidin kondensiert, dieses mittels Lithiumaluminiumhydrid zum 1-Benzyl-3,4-dihydroxypyrrolidin reduziert, aus diesem durch katalytische Hydrierung den Benzylrest abspaltet, das erhaltene 3,4-Dihydroxypyrrolidin mittels Di-tert.butyldicarbonat zum 1-tert.Butyloxycarbonyl-3,4-dihydroxypyrrolidin acyliert, dieses mittels Methylsulfonsäureanhydrid zum 1-tert.Butyloxycarbonyl-3,4-dimethylsulfonylpyrrolidin umsetzt, dieses mit Bromwasserstoff in Eisessiglösung in das 3,4-Dimethylsulfonylpyrrolidinhydrobromid umwandelt und schließlich dieses mit einem Alkalimetalldiphenylphosphid zum 3,4-Bis-(diphenylphosphino)-pyrrolidin umsetzt, welches als Hydrochlorid isoliert wird. Geht man bei dieser mehrstufigen Synthese von (+)-Weinsäure aus, erhält man das Hydrochlorid des (R,R)-3,4-Bis-(diphenylphosphino)-pyrrolidins, geht man von (—)-Weinsäure aus, entsteht in entsprechender Weise das Hydrochlorid des (S,S)-3,4-Bis-(diphenylphosphino)-pyrrolidins. Im Bedarfsfall kann dann aus dem Hydrochlorid durch Behandeln mit einem Alkalimetallhydroxid, -hydrogencarbonat oder -carbonat die entsprechende freie Base gewonnen werden.

Die freie Base kann anschließend mit Hilfe eines geeigneten Acylierungsmittels, insbesondere eines Carbonsäurechlorids oder -anhydrids, in an sich bekannter Weise am Stickstoffatom acyliert werden. Unter den Acylderivaten werden diejenigen besonders bevorzugt, für die in der Formel (I) R einen Benzoylrest, einen tert.Butyloxycarbonylrest oder einen Rest der Formel $CH_3—O—(CH_2—CH_2—O)_n—CH_2—CO—$, in der n für eine ganze Zahl von 0 bis 3 steht, bedeutet. Beispiele für weitere geeignete Acylreste sind Reste der Formel $R_1—CO—$, in der $R_1$ Wasserstoff, einen Methyl-, Ethyl-, n-Propyl-, i-Propyl-, n-Butyl-, i-Butyl-, sekundären Butyl- oder tertiären Butylrest oder einen α- oder β-Naphthylrest bedeutet. Dies sind bevorzugte Acylreste, doch sind die geeigneten Acylreste nicht allein auf diese beschränkt.

Die Acylreste können leicht in an sich bekannter Weise, z. B. durch Hydrierung der Carbonylgruppe mittels Lithiumaluminiumhydrid, in die entsprechenden Alkyl- bzw. Arylalkylreste überführt werden. Besonders bevorzugte Alkyl- bzw. Arylalkylderivate sind diejenigen, für die in der Formel (I) R einen Methyl-, Ethyl- oder Benzylrest bedeutet. Beispiele für andere geeignete Alkyl- bzw. Arylalkylsubstituenten am Pyrrolidin-Stickstoffatom sind n-Propyl-, i-Propyl-, n-Butyl-, i-Butyl-, n-Pentyl-, 2- und 3-Methylbutyl-2,2-Dimethylpropyl oder Naphthomethylreste. Aber auch andere Alkyl- bzw. Arylalkylreste sind geeignet.

Die so erhältlichen optisch aktiven Verbindungen der Formel (I) können als chirale Liganden in

Metallkomplexen dienen, die als Zentralatom Rhodium enthalten.

Ein weiterer Gegenstand der Erfindung sind daher Rhodiumkomplexe der Formel

$$[Rh(en)_2A]^+X^- \qquad (II)$$

in der $(en)_2$ zwei Moleküle eines Monoolefins oder ein Molekül eines Diolefins, A ein optisch aktives 3,4-Bis-(diphenylphosphino)-pyrrolidin der Formel (I) und $X^-$ ein Tetrafluoroborat-, Hexafluorophosphat- oder Perchloratanion bedeuten.

Für die Rhodiumkomplexe der Formel (II) geeignete Monoolefine sind beispielsweise Ethylen oder Cycloocten. Geeignete Diolefine sind beispielsweise 1,3-Butadien, 1,5-Cyclooctadien oder Norbornadien. Besonders bevorzugt sind Rhodiumkomplexe der Formel (II), die ein Molekül 1,5-Cyclooctadien enthalten.

Die Rhodiumkomplexe können hergestellt werden durch Umsetzung einer Verbindung der Formel (I) mit einem Rhodiumkomplex der Formel

$$[Rh(en)_2Y]_2 \qquad (III)$$

in der $(en)_2$ die bereits angegebene Bedeutung hat und Y Chlor, Brom oder Jod bedeutet, und einem Alkalimetall- oder Silbersalz der Tetrafluoroborsäure, Hexafluorophosphorsäure oder Perchlorsäure.

Die Rhodiumkomplexe der Formel (II), in denen $(en)_2$ ein Molekül 1,5-Cyclooctadien und $X^-$ ein Tetrafluoroboratanion bedeuten, können auf besonders einfache Weise auch dadurch hergestellt werden, daß man eine Verbindung der Formel (I) mit einem Rhodiumkomplex der Formel

$$[Rh(COD)_2]^+BF_4^- \qquad (IV)$$

in der COD für 1,5-Cyclooctadien steht, umsetzt.

Ein letzter Gegenstand der Erfindung ist schließlich die Verwendung der Rhodiumkomplexe der Formel (II) als Katalysatoren für die asymmetrische Hydrierung unsubstituierter oder β-substituierter α-Acylamino-acrylsäuren.

Als Substrate für die asymmetrische Hydrierung können beispielsweise α-Acylamino-acrylsäuren und deren Derivate der allgemeinen Formel

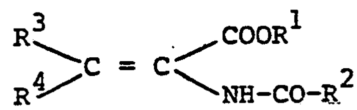

(V)

dienen. In dieser Formel bedeuten $R^1$ Wasserstoff, ein Alkalimetall oder einen niederen Alkylrest mit 1 bis 4, vorzugsweise 1 bis 2, Kohlenstoffatomen und $R^2$ einen niederen Alkylrest mit 1 bis 4, vorzugsweise 1 bis 2, Kohlenstoffatomen oder einen Phenylrest. $R^3$ und $R^4$ können gleich oder verschieden sein und Wasserstoff, einen geradkettigen oder verzweigten Alkylrest mit 1 bis 10 Kohlenstoffatomen, einen unsubstituierten oder in 3- und/oder 4-Stellung durch Hydroxyl-, Alkoxy- oder Acyloxygruppen substituierten Phenylrest oder einen unsubstituierten oder in 6-Stellung durch eine Methylgruppe oder durch Chlor substituierten Indolylrest bedeuten.

Durch die asymmetrische Hydrierung werden die genannten prochiralen Substrate in die entsprechenden optisch aktiven α-Acylaminocarbonsäuren umgewandelt, die sich in an sich bekannter Weise zu den entsprechenden α-Aminocarbonsäuren verseifen lassen. Wurde das im verwendeten Rhodiumkomplex enthaltene erfindungsgemäße optisch aktive 3,4-Bis-(diphenylphosphino)-pyrrolidin der Formel (I) ursprünglich aus (+)-Weinsäure hergestellt, so entsteht in großem Überschuß das L-Enantiomere der optisch aktiven α-Acylaminocarbonsäure, wurde dagegen von (—)-Weinsäure ausgegangen, so entsteht in großem Überschuß das D-Enantiomere. In beiden Fällen können die gewünschten Enantiomeren optisch nahezu vollständig rein erhalten werden.

Die Hydrierung erfolgt in den für Hydrierungen üblichen Lösungsmitteln, wie Alkoholen und Äthern oder Mischungen mit aliphatischen oder aromatischen Kohlenwasserstoffen oder mit Wasser. Die Substratkonzentration kann von einer 0,001-molaren bis zu einer an Substrat übersättigten Lösung reichen. Der Wasserstoffdruck kann zwischen Normaldruck und etwa 80 bar liegen, die Reaktionstemperatur zwischen — 20 und + 50 °C. Vorzugsweise wird die Hydrierung bei Raumtemperatur vorgenommen.

Die eine Verbindung der Formel (I) als chiralen Liganden enthaltenden Rhodiumkomplexe werden zweckmäßig in solcher Menge eingesetzt, daß das Molverhältnis von Substrat zu Katalysator im Bereich zwischen 1 : 1 und 50 000 : 1, vorzugsweise zwischen 500 : 1 bis 15 000 : 1, liegt.

Wegen der Empfindlichkeit der erfindungsgemäßen optisch aktiven 3,4-Bis-(diphenylphosphino)-pyrrolidine und der sie als chirale Liganden enthaltenden Rhodiumkomplexe gegenüber Sauerstoff ist es zweckmäßig, alle Reaktionen in Schutzgasatmosphäre, z. B. unter Stickstoff oder Argon, durchzuführen

und auch die jeweiligen Reaktionsprodukte unter Schutzgas aufzubewahren. Außerdem ist es empfehlenswert, auch die Hydrierungen unter anaeroben Bedingungen vorzunehmen.

Die Erfindung wird durch die nachfolgenden Beispiele näher erläutert, ohne daß dadurch der Umfang der Erfindung beschränkt werden soll.

## Beispiel 1

a) Herstellung von 1-Benzyl-2,5-dioxo-3,4-(R,R)-dihydroxypyrrolidin :

90 g (0,6 Mol) L-Weinsäure wurden mit 64,2 g (0,6 Mol) Benzylamin versetzt und unter Rühren auf 170 °C erhitzt. Das Reaktionswasser wurde dabei abgedampft. Nach ca. 30 Minuten wurde die braune Flüssigkeit fest und es wurde noch ca. 20 Minuten lang Vakuum angelegt. Nach dem Abkühlen wurde der noch warme Kristallbrei mit Ethanol digeriert und über Nacht stehen gelassen. Die Kristalle wurden abgesaugt, mit Ethanol nachgewaschen und aus Ethanol umkristallisiert. Ausbeute : 126 g, entsprechend 95 % der Theorie. Schmelzpunkt : 196 bis 198 °C (Literatur : 196 °C).

b) Herstellung von 1-Benzyl-3,4-(S,S)-dihydroxy-pyrrolidin :

12 g (0,32 Mol) Lithiumaluminiumhydrid wurden in 500 ml über KOH getrocknetem Diethylether unter Stickstoff gelöst. Zu der Lösung wurden bei Raumtemperatur auf einmal 15 g (0,068 Mol) des nach a) hergestellten 1-Benzyl-2,5-dioxo-3,4-(R,R)-dihydroxy-pyrrolidins zugegeben. Nach zweitägigem Rühren bei 35 °C wurde auf — 18 °C abgekühlt und es wurden langsam 12 ml Wasser zugetropft, wobei sich stürmisch Wasserstoff entwickelte. Anschließend wurden bei — 10 °C 12 ml 3,75 M-Natronlauge und schließlich bei 0 °C 25 ml Wasser zugetropft. Das Reaktionsgemisch wurde unter Rühren auf Raumtemperatur kommen gelassen, das Aluminiumhydroxid abfiltriert und in einem Soxhlet-Apparat 4 Tage lang mit dem Filtrat extrahiert.

Dann wurde der Ether im Rotationsverdampfer abgezogen und der Rückstand in 50 ml Essigester aufgenommen. Die nach Abkühlen auf — 18 °C ausgefallenen Kristalle wurden abgesaugt, mit Essigester/Ligroin (1 : 1) gewaschen und im Vakuum getrocknet. Ausbeute : 9,3 g (71 % der Theorie). Schmelzpunkt : 100 °C.

$[\alpha]_D^{RT} = +32{,}4°$ (c = 4,31, $CH_3OH$)
Elementaranalyse : $C_{11}H_{15}NO_2$ (193,25)
Berechnet : % C 68,36 % H 7,82 % N 7,25
Gefunden : % C 68,84 % H 7,87 % N 7,14

c) Herstellung von 3,4-(S,S)-Dihydroxy-pyrrolidin :

3,86 g (20 mMol) des nach b) hergestellten 1-Benzyl-3,4-(S,S)-dihydroxy-pyrrolidins wurden in 70 ml Ethanol gelöst, mit 0,5 g Palladium auf Aktivkohle (10 % Pd) versetzt und unter 1 bar Wasserstoffdruck hydriert. Nach Aufnahme der theoretischen Wasserstoffmenge wurde der Katalysator abfiltriert und das Filtrat im Vakuum zur Trockne eingedampft. Der Rückstand wurde im Hochvakuum sublimiert (Badtemperatur 100 °C, Druck $1{,}3 \cdot 10^{-4}$ mbar). Ausbeute : 1,93 g (94 % der Theorie). Schmelzpunkt : 101 bis 103 °C.

$[\alpha]_D^{RT} = +24{,}9°$ (c = 2,13, Ethanol)
Elementaranalyse : $C_4H_9NO_2$ (103,12)
Berechnet : % C 46,59 % H 8,80 % N 13,58
Gefunden : % C 46,84 % H 9,11 % N 13,58

d) Herstellung von 1-tert.Butyloxycarbonyl-3,4-(S,S)-dihydroxy-pyrrolidin :

4,30 g (41,7 mMol) des nach c) hergestellten 3,4-(S,S)-Dihydroxy-pyrrolidins wurden in 80 ml Ethanol gelöst und auf 0 °C gekühlt. Zu der Lösung wurden 10,34 g (47,4 mMol) Di-tert.butyl-dicarbonat zugegeben und es wurde über Nacht gerührt. Dann wurde 1 Stunde auf 50 °C erwärmt und anschließend das Lösungsmittel in Vakuum abgezogen. Der Rückstand wurde in 71 ml Essigester und 173 ml Toluol zum Sieden erhitzt und dann erkalten gelassen. Nach 12 Stunden stehen bei — 20 °C wurden die ausgeschiedenen Kristalle abgesaugt, zweimal mit Toluol nachgewaschen und getrocknet. Ausbeute : 7,30 g (86 % der Theorie). Schmelzpunkt : 163 bis 165 °C.

$[\alpha]_D^{RT} = -21{,}9°$ (c = 3,065, $CH_3OH$)
Elementaranalyse : $C_9H_{17}NO_4$ (203,24)
Berechnet : % C 53,19 % H 8,43 % N 6,89
Gefunden : % C 53,49 % H 8,31 % N 6,85
$^1H$-NMR ($CD_3OD$) δ (ppm) :
4,03, m, 2 H (2 × C<u>H</u>—OH)
3,20-3,62, m, 4 H (2 × C<u>H</u>$_2$)
1,46, s, 9 H (3 × C<u>H</u>$_3$)

e) Herstellung von 1-tert.Butyloxycarbonyl-3,4-(S,S)-dimethansulfonyl-pyrrolidin :

12,18 g (59,9 mMol) des nach d) hergestellten 1-tert.-Butyloxycarbonyl-3,4-(S,S)-dihydroxy-pyrrolidins wurden in 150 ml absolutem Chloroform gelöst. Die Lösung wurde mit 10,6 ml (131 mMol) absolutem Pyridin versetzt und auf — 50 °C gekühlt.

Dann wurden 25 g (143,5 mMol) Methansulfonsäureanhydrid auf einmal zugegeben und es wurde sofort auf — 60 °C heruntergekühlt. Das Reaktionsgemisch wurde langsam auf Raumtemperatur kommen gelassen und 24 Stunden lang nachgerührt. Die entstandene bräunliche Suspension wurde mit 300 ml Methylenchlorid versetzt und dreimal mit einem eiskalten Gemisch von je 50 ml $H_2O$ und 5 ml 2N-Salzsäure ausgeschüttelt. Danach wurde zweimal mit je 50 ml Wasser gewaschen und über $MgSO_4$ getrocknet. Das Lösungsmittel wurde im Vakuum abgezogen und der Rückstand im Hochvakuum getrocknet. Ausbeute : 22,23 g (100 % der Theorie) rohes Produkt, das noch Spuren von Chloroform und Methylenchlorid enthielt und ohne weitere Reinigung für die nächste Verfahrensstufe eingesetzt wurde.

f) Herstellung von 3,4-(S,S)-Dimethansulfonyl-pyrrolidin-hydrobromid :

22,23 g (59,9 mMol) des nach e) erhaltenen rohen 1-tert.Butyloxycarbonyl-3,4-(S,S)-dimethansulfonyl-pyrrolidins wurden unter leichtem Erwärmen in 250 ml Essigester gelöst. Die geringen ungelösten Anteile wurden abfiltriert und mit 50 ml Essigester nachgewaschen. Die vereinigten Filtrate wurden auf — 10 °C gekühlt und mit 18 ml einer HBr-Lösung in Eisessig (40 % HBr) versetzt. Nach 30 Sekunden begann ein weißer Niederschlag auszufallen. Das Reaktionsgemisch wurde auf Raumtemperatur kommen gelassen und 24 Stunden lang nachgerührt. Dann wurde der Niederschlag abgesaugt, unter Stickstoff dreimal mit absolutem Ether gewaschen und im Hochvakuum getrocknet. Ausbeute : 19,11 g (94 % der Theorie).

Elementaranalyse : $C_6H_{14}BrNO_6S_2$ (340,22)
Berechnet : % C 21,18 % H 4,15 % N 4,12 % S 18,85
Gefunden : % C 21,80 % H 4,11 % N 4,27 % S 18,52

g) Herstellung von 3,4-(R,R)-Bis-(diphenylphosphino)-pyrrolidin-hydrochlorid :

10,56 g (27,5 mMol) Natriumdiphenylphosphid. 2 Dioxan wurden in 60 ml absolutem Dimethylformamid gelöst. Die Lösung wurde auf — 35 °C gekühlt und auf einmal mit 2,6 g (7,6 mMol) des nach f) hergestellten 3,4-(S,S)-Dimethansulfonyl-pyrrolidin-hydrobromids versetzt. Dann wurde die Badtemperatur auf — 12 °C erhöht und es wurde 15 Stunden lang gerührt. Das Reaktionsgemisch wurde auf Raumtemperatur kommen gelassen und das Lösungsmittel wurde bei einer Badtemperatur von 20 °C im Hochvakuum abgezogen. Der rote Rückstand wurde mit 50 ml entgastem Wasser versetzt und die wäßrige Lösung einmal mit 35 ml und zweimal mit 15 ml Ether extrahiert. Die vereinigten Etherphasen wurden mit 40 ml 0,8M-Salzsäure versetzt und 15 Stunden lang kräftig gerührt. Das ausgefallene Hydrochlorid wurde abgesaugt, mit Wasser und Ether nachgewaschen und bei 50 °C im Hochvakuum getrocknet. Ausbeute : 3,12 g (80 % der Theorie). Schmelzpunkt : 184 bis 186 °C.

$[\alpha]_D^{RT}$ = + 170° (c = 2,8, Ethanol 99 %)
Elementaranalyse : $C_{28}H_{28}ClNP_2$ (475,94)
Berechnet : % C 70,66 % H 5,93 % N 2,94
Gefunden : % C 70,55 % H 6,09 % N 2,83
$^{31}$P-NMR ($CD_3OD/CH_3OH$) δ (ppm) : — 15,28, s
$^1$H-NMR ($CD_3OD$) δ (ppm) : 7,6-7,1, m, 20 H
4,1-3,6, m, 2 H
3,1, m, 4 H

h) Herstellung von 3,4-(R,R)-Bis-(diphenylphosphino)-pyrrolidin :

0,708 g (1,49 mMol) des nach g) hergestellten 3,4-(R,R)-Bis-(diphenylphosphino)-pyrrolidin-hydrochlorids wurden unter Stickstoff in 20 ml Wasser, das 230 mg (4,1 mMol) KOH enthielt, 1 Stunde gerührt. Dann wurde dreimal mit je 20 ml Ether extrahiert und die vereinigten Etherphasen wurden über $MgSO_4$ getrocknet und im Hochvakuum vom Ether befreit. Ausbeute : 0,644 g (98,3 % der Theorie).

Elementaranalyse : $C_{28}H_{27}NP_2$ (439,48)
Berechnet : % C 76,52 % H 6,19 % N 3,19
Gefunden : % C 76,67 % H 6,13 % N 3,21
$^1$H-NMR ($CD_2Cl_2$) δ (ppm) : 7,08-7,56, m, 20 H (Phenyl)
3,12-3,50, m, 2 H (P—C—$\underline{H}$)
1,83 (breit), 1 H (N—$\underline{H}$)
2,73-3,00, m, 4 H (C—C$\underline{H_2}$—N)

$[\alpha]_D^{RT}$ = + 153° (c = 2,35, Ethanol 99 %)

## Beispiel 2

Herstellung von [(3,4-(R,R)-Bis-(diphenylphosphino)-pyrrolidin)(COD)Rh]$BF_4$ :

574 mg (1,45 mMol) $[Rh(COD)_2]BF_4$ wurden in 5 ml absolutem Methylenchlorid gelöst. Die Lösung wurde auf —30 °C abgekühlt und mit einer Lösung von 638 mg (1,45 mMol) des nach Beispiel 1h) hergestellten 3,4-(R,R)-Bis-(diphenylphosphino)-pyrrolidins in 20 ml absolutem Methylenchlorid versetzt. Nach 5 Minuten Rühren bei —30 °C wurde das Kühlbad entfernt und nach Erreichen von Raumtemperatur wurde im Hochvakuum auf ca. 5 ml eingeengt. Durch Zugabe von 25 ml absolutem Ether fiel der Rhodiumkomplex als gelbes Pulver aus. Ausbeute : 1,048 g (98,03 % der Theorie).

Elementaranalyse : $C_{36}H_{39}NP_2RhBF_4$ (737,37)

Berechnet : % C 58,64 % H 5,33 % N 1,89

Gefunden : % C 58,67 % H 5,51 % N 1,86

$^{31}P$-NMR ($CD_2Cl_2/CH_2Cl_2$) δ (ppm) : 28,17, d

$^{1}J_{Rh-P}$ = 148 Hz

## Beispiel 3

Hydrierungen mit dem gemäß dem Beispiel 2 hergestellten Rhodiumkomplex :

a) In einen 0,5 l-Stahlautoklaven, ausgerüstet mit einem Magnethubrührer, wurden 51,3 g α-Acetamidozimtsäure eingewogen. Der Autoklav wurde verschlossen und evakuiert. Dann wurden 23 mg des Rhodiumkomplexes als Lösung in 250 ml absolutem Methanol in den Autoklaven eingesaugt. Nach Durchspülen mit $H_2$ wurde $H_2$ bis zu einem Druck von 60 bar aufgepreßt, der Autoklav auf 50 °C erwärmt und der Rührer in Gang gesetzt. Nach 20 Stunden war die $H_2$-Aufnahme beendet und der Druck auf 40 bar abgefallen. Der Autoklav wurde entleert und das Methanol wurde abgezogen. Zur Entfernung des Katalysators wurde der Rückstand mit Chloroform ausgewaschen und anschließend getrocknet. Umsatz : 100 %

Ausbeute an N-Acetyl-L-phenylalanin : 51,2 g (98,8 % der Theorie).

Schmelzpunkt : 169 °C

$[\alpha]_D^{RT}$ = + 45,5° (c = 1, Ethanol 95 %), entsprechend einer optischen Ausbeute von 95,8 % bei einem Bezugswert $[\alpha]_D^{RT}$ = + 47,5°.

b) Analog zu a) wurden in einem 0,1 l-Stahlautoklaven 1,04 g α-Acetamidozimtsäure in Gegenwart von 6,3 mg des Rhodiumkomplexes hydriert.

Lösungsmittel : 30 ml Methanol

$H_2$-Anfangsdruck : 50 bar

Hydriertemperatur : Raumtemperatur

Hydrierzeit : 15 Stunden

Umsatz : 100 %

Optische Ausbeute : 97,5 %

## Beispiel 4

Herstellung von 1-Benzoyl-3,4-(R,R)-bis-(diphenylphosphino)-pyrrolidin :

559 mg (1,1 mMol) des nach Beispiel 1g) hergestellten 3,4-(R,R)-Bis-(diphenylphosphino)-pyrrolidin-hydrochlorids wurden unter Stickstoff in 15 ml Wasser suspendiert und mit einer Lösung von 1,5 g (27 mMol) Kaliumhydroxid in 10 ml Wasser versetzt. Die freie Base wurde zweimal mit je 25 ml Toluol extrahiert. Die vereinigten Extrakte wurden über Magnesiumsulfat getrocknet. Nach Filtration wurden 1 ml (7 mMol) Triethylamin und anschließend 0,27 ml (2,3 mMol) Benzoylchlorid zugegeben. Nach einer Stunde Rühren wurden 50 ml Wasser zugesetzt und die Phasen wurden getrennt. Die organische Phase wurde mit 0,5M-Natronlauge, Wasser, einer 10 %igen wäßrigen Lösung von $NaH_2PO_4$ und schließlich wieder mit Wasser gewaschen. Die Toluollösung wurde über $MgSO_4$ getrocknet, auf 10 ml eingeengt und durch Zugabe von n-Hexan wurde das 1-Benzoyl-3,4-(R,R)-bis-(diphenylphosphino)-pyrrolidin ausgefällt. Dieses wurde abgesaugt, mit n-Hexan gewaschen und im Hochvakuum getrocknet. Ausbeute 527 mg (89 % der Theorie). Schmelzpunkt : 180 bis 182 °C (im Hochvakuum eingeschmolzen).

Elementaranalyse : $C_{35}H_{31}NOP_2$ (543,59)

Berechnet : % C 77,34 % H 5,75 % N 2,58

Gefunden : % C 77,73 % H 5,29 % N 2,63

$[\alpha]_D^{RT}$ = + 153° (c = 2,84, Toluol)

## Beispiel 5

Herstellung von [(1-Benzoyl-3,4-(R,R)-bis-(diphenylphosphino)-pyrrolidin)(COD)Rh]$BF_4$ :

0,20 g (0,5 mMol) $[Rh(COD)_2]BF_4$ und 0,272 g des nach Beispiel 4 hergestellten 1-Benzoyl-3,4-(R,R)-

bis-(diphenylphosphino)-pyrrolidins wurden in 25 ml Methylenchlorid 2 Stunden lang gerührt. Dann wurde im Vakuum auf 2 ml eingeengt, auf —30 °C gekühlt und ein Gemisch aus Tetrahydrofuran und Diethylether (Volumenverhältnis 1 : 2) zugesetzt. Der gelbe Niederschlag wurde bei Raumtemperatur abgesaugt und mit Ether gewaschen. Anschließend wurde der Rhodiumkomplex aus Methanol mit Ether umgefällt. Ausbeute : 0,383 g (91 % der Theorie).

Elementaranalyse : $C_{43}H_{43}NOP_2RhBF_4$ (841,48)
Berechnet : % C 61,38 % H 5,15 % N 1,66
Gefunden : % C 61,94 % H 5,06 % N 1,56

Beispiel 6

Hydrierungen mit dem gemäß Beispiel 5 hergestellten Rhodiumkomplex :

Die Hydrierungen wurden analog Beispiel 3 durchgeführt.

a) Reaktionsgefäß : 0,5 l-Stahlautoklav
Eingesetztes Substrat : 20,5 g α-Acetamidozimtsäure
Einsatzmenge an Rhodiumkomplex : 46 mg
Lösungsmittel : 200 ml Methanol
$H_2$-Anfangsdruck : 20 bar
Hydriertemperatur : 25 bis 28 °C
Hydrierzeit : 2 Stunden
Umsatz : 100 %
Optische Ausbeute : 97,5 %

b) Reaktionsgefäß : 0,5 l-Stahlautoklav
Eingesetzes Substrat : 102,5 g α-Acetamidozimtsäure
Einsatzmenge an Rhodiumkomplex : 50 mg
Lösungsmittel : 260 ml Methanol
$H_2$-Anfangsdruck : 55 bar
Hydriertemperatur : 22 °C
Hydrierzeit : 14 Stunden
$H_2$-Enddruck : 45 bar
Umsatz : 100 %
Optische Ausbeute : 98,9 %

c) Reaktionsgefäß : 0,1 l-Erlenmeyerkolben mit Magnetrührer und Gaseinleitungsvorrichtung
Eingesetztes Substrat : 1,15 g α-Acetamidozimtsäure
Einsatzmenge an Rhodiumkomplex : 29 mg
Lösungsmittel : 10 ml Methanol
$H_2$-Druck : 1 bar (aus Gasometer)
Hydriertemperatur : 50 °C
Hydrierzeit : 4 Stunden
Umsatz : 100 %
Optische Ausbeute : 95,0 %

d) Reaktionsgefäß : 0,1 l-Stahlautoklav
Eingesetztes Substrat : 2,05 g α-Acetamidozimtsäure
Einsatzmenge an Rhodiumkomplex : 1 mg
Lösungsmittel : 30 ml Methanol
$H_2$-Anfangsdruck : 47 bar
Hydriertemperatur : Raumtemperatur
Hydrierzeit : 19 Stunden
Umsatz : 100 %
Optische Ausbeute : 99,0 %

e) Reaktionsgefäß : 0,1 l-Stahlautoklav
Eingesetztes Substrat : 3,25 g α-Acetamidozimtsäure
Einsatzmenge an Rhodiumkomplex : 1,3 mg
Lösungsmittel : 25 ml Methanol
$H_2$-Anfangsdruck : 60 bar
Hydriertemperatur : Raumtemperatur
Hydrierzeit : 20 Stunden
Umsatz : 98 %
Optische Ausbeute : 96,0 %

f) Reaktionsgefäß : 0,1 l-Stahlautoklav
Eingesetztes Substrat : 2,1 g α-Acetamidozimtsäure
Einsatzmenge an Rhodiumkomplex : 9,9 mg
Lösungsmittel : 25 ml luftgesättigtes Methanol

$H_2$-Anfangsdruck : 47 bar
Hydriertemperatur : Raumtemperatur
Hydrierzeit : 0,5 Stunden
Umsatz : 100 %
Optische Ausbeute : 98,0 %

g) Reaktionsgefäß : 0,1 l-Erlenmeyerkolben mit Magnetrührer und Gaseinleitungsvorrichtung
Eingesetztes Substrat : 1,28 g α-Benzamidozimtsäure
Einsatzmenge an Rhodiumkomplex : 17,8 mg
Lösungsmittel : 15 ml Tetrahydrofuran
$H_2$-Druck : 1 bar (aus Gasometer)
Hydriertemperatur : Raumtemperatur
Hydrierzeit : 40 Stunden
Produkt : N-Benzoyl-L-phenylalanin
Umsatz : 100 %
Optische Ausbeute : 94,4 %

h) Reaktionsgefäß : 0,1 l-Stahlautoklav
Eingesetzes Substrat : 1,1 g α-Acetamido-β-(4-hydroxyphenyl)-acrylsäure
Einsatzmenge an Rhodiumkomplex : 5,0 mg
Lösungsmittel : 50 ml Methanol
$H_2$-Anfangsdruck : 65 bar
Hydriertemperatur : Raumtemperatur
Hydrierzeit : 24 Stunden
Produkt : N-Acetyl-L-tyrosin
Umsatz : 100 %
Optische Ausbeute : 97,0 %

i) Reaktionsgefäß : 0,1 l-Stahlautoklav
Eingesetztes Substrat : 1,25 g α-Acetamido-β-(3-methoxy-4-hydroxyphenyl)-acrylsäure
Einsatzmenge an Rhodiumkomplex : 5,0 mg
Lösungsmittel : 50 ml Methanol
$H_2$-Anfangsdruck : 65 bar
Hydriertemperatur : Raumtemperatur
Hydrierzeit : 24 Stunden
Produkt : N-Acetyl-L-(3-methoxy-4-hydroxyphenyl)-alanin
Umsatz : 100 %
Optische Ausbeute : 100 %

j) Reaktionsgefäß : 0,1 l-Stahlautoklav
Eingesetztes Substrat : 1,1 g α-Acetamido-β-(4-methoxyphenyl)-acrylsäure
Einsatzmenge an Rhodiumkomplex : 5,0 mg
Lösungsmittel : 50 ml Methanol
$H_2$-Anfangsdruck : 65 bar
Hydriertemperatur : Raumtemperatur
Hydrierzeit : 15 Stunden
Produkt : N-Acetyl-L-(4-methoxyphenyl)-alanin
Umsatz : 80 %
Optische Ausbeute : 100 % (auf den Umsatz korrigiert)

k) Reaktionsgefäß : 0,1 l-Stahlautoklav
Eingesetzes Substrat : 1,0 mg α-Acetamido-β-(3,4-methylendioxyphenyl)-acrylsäure
Einsatzmenge an Rhodiumkomplex : 5,0 mg
Lösungsmittel : 50 ml Methanol
$H_2$-Anfangsdruck : 55 bar
Hydriertemperatur : Raumtemperatur
Hydrierzeit : 15 Stunden
Produkt : N-Acetyl-L-(3,4-methylendioxyphenyl)-alanin
Umsatz : 100 %
Optische Ausbeute : 93,0 %

## Beispiel 7

Herstellung von 1-tert.-Butyloxycarbonyl-3,4-(R,R)-bis-(diphenylphosphino)-pyrrolidin :

0,78 g (1,64 mMol) des nach Beispiel 1g) hergestellten 3,4-(R,R)-Bis-(diphenylphosphino)-pyrrolidin-hydrochlorids wurden in 20 ml Chloroform gelöst. Dann wurde unter Stickstoff eine Lösung von 0,23 g (2,73 mMol) Natriumhydrogencarbonat und 0,41 g (7,01 mMol) Kochsalz in 20 ml Wasser zugegeben.

Nach Beendigung der Gasentwicklung wurde eine Lösung von 0,40 g (1,83 mMol) Di-tert.butyldicarbonat in 10 ml Chloroform zugesetzt und 1,5 Stunden lang bei 50 °C gerührt. Nach Abkühlen auf

Raumtemperatur wurde weitere 12 Stunden lang gerührt. Anschließend wurde die organische Phase abgetrennt und das Chloroform abgezogen. Der Rückstand wurde in 10 ml Diethylether aufgenommen und das 1-tert.Butyloxycarbonyl-3,4-(R,R)-bis-(diphenylphosphino)-pyrrolidin durch Zugabe eines Gemisches aus 10 ml Methanol und 5 ml Wasser als gelartiger weißer Niederschlag ausgefällt. Das Produkt wurde abgesaugt und im Hochvakuum getrocknet.

Elementaranalyse : $C_{33}H_{35}NO_2P_2$ (539,58)
Berechnet : % C 73,45 % H 6,53 % N 2,59
Gefunden : % C 73,22 % H 6,81 % N 2,57
$^{31}$P-NMR ($CD_2Cl_2/CH_2Cl_2$) δ (ppm) : — 11,63, s
$^{1}$H-NMR ($d_6$-Aceton) δ (ppm) : 7,24-7,55, m, 20 H
2,93-4,04, mehrere m, 6 H, alle Protonen des Rings
1,38, s, 9 H, —$C\underline{H}_3$
IR : ν(C = 0) : 1 692 $cm^{-1}$
$[\alpha]_D^{RT}$ = + 125° (c = 3,6, Toluol)

## Beispiel 8

Herstellung von [(1-tert.Butyloxycarbonyl-3,4-(R,R)-bis-(diphenylphosphino)-pyrrolidin)(COD)Rh]$BF_4$ :

Zu einer Lösung von 249 mg (0,61 mMol) $[Rh(COD)_2]BF_4$ in 10 ml absolutem Methylenchlorid wurden bei — 20 °C 338 mg (0,63 mMol) des nach Beispiel 7 hergestellten 1-tert.Butyloxycarbonyl-3,4-(R,R)-bis-(diphenylphosphino)-pyrrolidins zugegeben und das Reaktionsgemisch wurde bei Raumtemperatur über Nacht gerührt. Nach Abziehen des Lösungsmittels wurde das Produkt in 5 ml Methanol aufgenommen und der Rhodiumkomplex durch Zugabe von 10 ml Diethylether ausgefällt. Ausbeute : 456 mg (89,4 % der Theorie).

Elementaranalyse : $C_{41}H_{47}NO_2P_2RhBF_4$ (837,49)
Berechnet : % C 58,79 % H 5,65 % N 1,67
Gefunden : % C 58,55 % H 5,67 % N 1,53
$^{31}$P-NMR ($CD_2Cl_2/CH_2Cl_2$) δ (ppm) : 33,23
$^{1}J_{Rh-P}$ = 149,9 Hz

## Beispiel 9

Hydrierungen mit dem gemäß Beispiel 8 hergestellten Rhodiumkomplex :

Die Hydrierungen wurden analog Beispiel 3 durchgeführt.

a) Reaktionsgefäß : 0,5 l-Stahlautoklav
Eingesetztes Substrat : 51,3 g α-Acetamidozimtsäure
Einsatzmenge an Rhodiumkomplex : 26,2 mg
Lösungsmittel : 250 ml Methanol
$H_2$-Anfangsdruck : 60 bar
Hydriertemperatur : 17 °C
Hydrierzeit : 17 Stunden
$H_2$-Enddruck : 40 bar
Umsatz : 100 %
Optische Ausbeute : 97,7 %

b) Reaktionsgefäß : 0,1 l-Stahlautoklav
Eingesetzes Substrat : 1,03 g α-Acetamidozimtsäure
Einsatzmenge an Rhodiumkomplex : 4,0 mg
Lösungsmittel : 25 ml Methanol
$H_2$-Anfangsdruck : 50 bar
Hydriertemperatur : Raumtemperatur
Hydrierzeit : 20 Stunden
Umsatz : 100 %
Optische Ausbeute : 99,6 %

c) Reaktionsgefäß : 0,1 l-Stahlautoklav
Eingesetztes Substrat : 1,44 g α-Benzamido-β-(3-indolyl)-acrylsäure
Einsatzmenge an Rhodiumkomplex : 3,5 mg
Lösungsmittel : 25 ml Methanol (Substrat liegt in Suspension vor)
$H_2$-Anfangsdruck : 50 bar
Hydriertemperatur : Raumtemperatur
Hydrierzeit : 20 Stunden
Produkt : N-Benzoyl-L-tryptophan

Umsatz : 100 %
Optische Ausbeute : 81,0 %

d) Reaktionsgefäß : 0,1 l-Stahlautoklav
Eingesetztes Substrat : 1,73 g α-Acetamido-β-(3-methoxy-4-hydroxyphenyl)-acrylsäure
Einsatzmenge an Rhodiumkomplex : 5,8 mg
Lösungsmittel : 25 ml Methanol
$H_2$-Anfangsdruck : 50 bar
Hydriertemperatur : Raumtemperatur
Hydrierzeit : 20 Stunden
Produkt : N-Acetyl-L-(3-methoxy-4-hydroxyphenyl)-alanin
Umsatz : 100 %
Optische Ausbeute : über 97 %

## Beispiel 10

Herstellung von 1-(β-Methoxyethoxyacetyl)-3,4-(R,R)-bis-(diphenylphosphino)-pyrrolidin :

Zu einer Lösung von 0,65 g (1,36 mMol) des nach Beispiel 1g) hergestellten 3,4-(R,R)-Bis-(diphenylphosphino)-pyrrolidin-hydrochlorids in 20 ml absolutem Methylenchlorid wurden 0,23 ml (2,8 mMol) Pyridin zugegeben. Nach Abkühlen auf —40 °C wurden 0,52 g (3,4 mMol) (β-Methoxyethoxy)-essigsäurechlorid zugesetzt. Das Reaktionsgemisch wurde auf Raumtemperatur kommen gelassen und über das Wochenende gerührt. Durch Zugabe von Diethylether wurde das 1-(β-Methoxyethoxyacetyl)-3,4-(R,R)-bis-(diphenylphosphino)-pyrrolidin als Öl ausgefällt, das bei viertägigem Rühren in Diethylether in ein weißes Pulver überging.

Das Rohprodukt wurde in Methylenchlorid gelöst und mit 2N-HCl extrahiert. Das Methylenchlorid wurde abgezogen, der Rückstand in Ethanol aufgenommen und mit Wasser ausgefällt. Die sehr hygroskopische Substanz wurde tagelang im Hochvakuum getrocknet. Ausbeute : 75 % der Theorie.

Elementaranalyse : $C_{33}H_{35}NO_3P222 \cdot 0,5\ H_2O$ (564,61)
Berechnet : % C 70,20 % H 6,43 % N 2,48
Gefunden : % C 70,08 % H 6,43 % N 2,41
$^{31}P$-NMR ($CD_2Cl_2$) δ (ppm) : —10,17, s
$^1H$-NMR ($CD_2Cl_2$) δ (ppm) :
7,13-7,60, m, 20 H, Phenyle
3,98, s, 2 H, > N—CO$CH_2$—O—
3,31, s, 3 H, —O$CH_3$
3,0-4,1, mehrere m, 15 H, alle Protonen außer Phenyl

$[\alpha]_D^{RT} = +111 \pm 10°$ (c = 2,9, Toluol)

## Beispiel 11

Herstellung von [(1-(β-Methoxyethoxyacetyl)-3,4-(R,R)-bis-(diphenylphosphino)-pyrrolidin)(COD)Rh]$BF_4$

Zu einer Lösung von 398 mg (0,98 mMol) $[Rh(COD)_2]\ BF_4$ in 10 ml absolutem Methylenchlorid wurden bei —30 °C 561 mg (1,00 mMol) des nach Beispiel 10 herstellten 1-(β-Methoxyethoxyacetyl)-3,4-(R,R)-bis-(diphenylphosphino)-pyrrolidins als Lösung in 10 ml Methylenchlorid zugetropft. Nach Rühren bei Raumtemperatur über Nacht wurde das Methylenchlorid im Hochvakuum abgezogen, der Rückstand in 10 ml Methanol aufgenommen und der Rhodiumkomplex durch Zugabe von Diethylether ausgefällt. Nach Abzentrifugieren wurde der Komplex mit Diethylether gewaschen und im Hochvakuum getrocknet. Der Komplex wurde durch $^{31}P$-NMR und $^1H$-NMR charakterisiert :

$^{31}P$-NMR ($CD_2Cl_2$) δ (ppm) : 35,23, d, $J_{Rh-P} = 149,1$ Hz
35,44, d, $J_{Rh-P} = 149,6$ Hz
$^1H$-NMR ($CD_2Cl_2$) δ (ppm) :
7,60-8,04, m, Phenyle
5,2 und 4,6, breit, olefinische Protonen von COD
3,85, s, > N—CO—$CH_2$—O—
3,41, m, —O—$CH_2$—$CH_2$—O—
3,17, s, —O—$CH_3$
2,15-3,85, mehrere m, alle Ringprotonen und nichtolefinischen COD-Protonen

## Beispiel 12

Hydrierungen mit dem gemäß Beispiel 11 hergestellten Rhodiumkomplex :

Die Hydrierungen wurden analog Beispiel 3 durchgeführt

a) Reaktionsgefäß : 0,5 l-Stahlautoklav
Eingesetztes Substrat : 51,3 g α-Acetamidozimtsäure
Einsatzmenge an Rhodiumkomplex : 26,7 mg
Lösungsmittel : 250 ml Methanol
$H_2$-Anfangsdruck : 60 bar
Hydriertemperatur : 17 °C
Hydrierzeit : 17 Stunden
$H_2$-Enddruck : 40 bar
Umsatz : 100 %
Optische Ausbeute : 96,8 %

b) Reaktionsgefäß : 0,1 l-Stahlautoklav
Eingesetztes Substrat : 1,0 g α-Acetamidozimtsäure
Einsatzmenge an Rhodiumkomplex : 4,2 mg
Lösungsmittel : 25 ml Methanol
$H_2$-Anfangsdruck : 50 bar
Hydriertemperatur : Raumtemperatur
Hydrierzeit : 20 Stunden
Umsatz : 100 %
Optische Ausbeute : 98,7 %

Beispiel 13

Herstellung von 1-(β-Methoxyethoxyethoxyethoxyacetyl)-3,4-(R,R)-bis-(diphenylphosphino)-pyrrolidin :

Zu einer Lösung von 1,04 g (2,18 mMol) des nach Beispiel 1g) hergestellten 3,4-(R,R)-Bis-(diphenylphosphino)-pyrrolidin-hydrochlorids in 20 ml absolutem Methylenchlorid wurden 0,35 ml (4,36 mMol) Pyridin zugegeben. Die Lösung wurde auf — 35 °C abgekühlt und es wurden 0,55 g (2,22 mMol) (β-Methoxyethoxyethoxyethoxy)-essigsäurechlorid zugesetzt. Nach Erwärmen auf Raumtemperatur und Rühren über Nacht wurde das Lösungsmittel abgezogen, der Rückstand in 10 ml Ethanol aufgenommen und das 1-(β-Methoxyethoxyethoxyethoxyacetyl)-3,4-(R,R)-bis-(diphenylphosphino)-pyrrolidin mit n-Pentan als Öl abgeschieden. Dieses Öl wurde abgetrennt, erneut mit n-Pentan versetzt, bei — 40 °C gerührt und schließlich eine Woche bei — 25 °C stehen gelassen. Da keine Kristallisation des zähen Öls erfolgte, wurde das n-Pentan abgegossen und der Rückstand im Hochvakuum getrocknet.

Das Rohprodukt wurde in Methylenchlorid gelöst und mit 2 N HCl extrahiert. Das Methylenchlorid wurde abgezogen, der Rückstand in Ethanol aufgenommen und mit Wasser ausgefällt. Die sehr hygroskopische Substanz wurde tagelang im Hochvakuum getrocknet. Ausbeute: 96 % der Theorie. Das Produkt wurde durch $^{31}P$-NMR und $^1H$-NMR charakterisiert :

$^{31}P$-NMR ($CH_2Cl_2$) δ (ppm) : — 10,17

$^1H$-NMR ($CD_2Cl_2$) δ (ppm) :
7,10-7,52, m, 20 H, Phenyle
3,99, s, 2 H, > N—CO—$C\underline{H}_2$—
3,59, schmal, 12 H, —O—$C\underline{H}_2$—$C\underline{H}_2$—O—
3,35, s, 3 H, —O—$C\underline{H}_3$
2,99-4,19, m, alle Protonen außer Phenyl

Beispiel 14

Herstellung von [(1)-(β-Methoxyethoxyethoxyethoxyacetyl)-3,4-(R,R)-bis-(diphenylphosphino)-pyrrolidin)(COD)Rh] $BF_4$ :

Zu einer Lösung von 362 mg (0,89 mMol) $[Rh(COD)_2]BF_4$ in 10 ml Methylenchlorid wurde bei — 30 °C eine Lösung von 542 mg (0,84 mMol) des nach Beispiel 13 hergestellten 1-(β-Methoxyethoxyethoxyethoxyacetyl)-3,4-(R,R)-bis-(diphenylphosphino)-pyrrolidins in 5 ml Methylenchlorid und 5 ml Ethanol zugegeben und das Reaktionsgemisch wurde bei Raumtemperatur über Nacht gerührt. Dabei entstanden geringe Mengen eines weißen Niederschlags, die abfiltriert wurden. Aus dem Filtrat wurde der Rhodiumkomplex durch Zugabe von Diethylether ausgefällt. Ausbeute : 79 % der Theorie. Der Komplex wurde durch $^{31}P$-NMR und $^1H$-NMR charakterisiert :

$^{31}P$-NMR ($CD_2Cl_2$) δ (ppm) :
33,59, d, $J_{Rh-P}$ = 149,6 Hz
33,27, d, $J_{Rh-P}$ = 149,0 Hz

$^1H$-NMR ($CD_2Cl_2$) δ (ppm) :
7,43-7,95, m, Phenyle
5,1 und 4,5, breit, olefinische Protonen von COD
3,77, s, —CO—$C\underline{H}_2$
3,39, m, —O—$CH_2$—$C\underline{H}_2$—O—
3,20, s, —O—$C\underline{H}_3$
1,68-3,77, mehrere m, alle Ringprotonen und nichtolefinischen COD-Protonen

Beispiel 15

Hydrierungen mit dem gemäß Beispiel 14 hergestellten Rhodiumkomplex :

Die Hydrierungen wurden analog Beispiel 3 durchgeführt.

a) Reaktionsgefäß : 0,5 l-Stahlautoklav
Eingesetztes Substrat : 51,3 g α-Acetamidozimtsäure
Einsatzmenge an Rhodiumkomplex : 29,4 mg
Lösungsmittel : 250 ml Methanol
$H_2$-Anfangsdruck : 60 bar
Hydriertemperatur : 50 °C
Hydrierzeit : 18 Stunden
$H_2$-Enddruck : 40 bar
Umsatz : 100 %
Optische Ausbeute : 96,4 %

b) Reaktionsgefäß : 0,5 l-Stahlautoklav
Eingesetztes Substrat : 51,3 g α-Acetamidozimtsäure
Einsatzmenge an Rhodiumkomplex : 14,7 mg
Lösungsmittel : 250 ml Methanol
$H_2$-Anfangsdruck : 60 bar
Hydriertemperatur : 50 °C
Hydrierzeit : 24 Stunden
$H_2$-Enddruck : 40 bar
Umsatz : 89 %
Optische Ausbeute : 98,4 % (auf den Umsatz korrigiert)

c) Reaktionsgefäß : 0,1 l-Stahlautoklav
Eingesetztes Substrat : 1,0 g α-Acetamidozimtsäure
Einsatzmenge an Rhodiumkomplex : 3,4 mg
Lösungsmittel : 25 ml Methanol
$H_2$-Anfangsdruck : 50 bar
Hydriertemperatur : Raumtemperatur
Hydrierzeit : 20 Stunden
Umsatz : 100 %
Optische Ausbeute : 97,7 %

Beispiel 16

Herstellung von 1-Benzyl-3,4-(R,R)-bis-(diphenylphosphino)-pyrrolidin :

Eine Lösung von 544 mg (1 mMol) des nach Beispiel 4 hergestellten 1-Benzoyl-3,4-(R,R)-bis-(diphenylphosphino)-pyrrolidins in 25 ml absolutem Tetrahydrofuran wurde zu einer Lösung von 1 g (0,025 mMol) Lithiumaluminiumhydrid in 25 ml absolutem Tetrahydrofuran zugetropft und das Reaktionsgemisch wurde über Nacht gerührt. Dann wurden 1 ml Wasser, 1 ml 3,75 M-Natronlauge und schließlich weitere 3 ml Wasser zugetropft. Das Reaktionsgemisch wurde filtriert und aus dem Filtrat wurde das Lösungsmittel im Vakuum abdestilliert. Der Rückstand wurde in Toluol aufgenommen und das N-Benzylderivat durch Zugabe von n-Pentan ausgefällt, abgesaugt und getrocknet. Ausbeute : 0,43 g (81 % der Theorie).

Elementaranalyse : $C_{35}H_{33}NP_2$ (529,60)
Berechnet : % C 79,38 % H 6,28 % N 2,64
Gefunden : % C 79,61 % H 6,35 % N 2,59

Beispiel 17

Herstellung von [(1-Benzyl-3,4-(R,R)-bis-(diphenylphosphino)-pyrrolidin)(COD)Rh]$BF_4$ :

Eine Lösung von 0,20 g (0,5 mMol) [Rh(COD)$_2$]$BF_4$ und 0,265 g (0,5 mMol) des nach Beispiel 16 hergestellten 1-Benzyl-3,4-(R,R)-bis-(diphenylphosphino)-pyrrolidins in 25 ml Methylenchlorid wurde 2 Stunden lang gerührt. Dann wurde im Vakuum auf 2 ml eingeengt und der Rhodiumkomplex wurde durch Zugabe von Diethylether ausgefällt, abgesaugt und getrocknet. Ausbeute : 0,376 g (91 % der Theorie).

Elementaranalyse : $C_{43}H_{45}NP_2RhBF_4$ (827,5)
Berechnet : % C 62,41 % H 5,48 % N 1,69
Gefunden : % C 62,38 % H 5,55 % N 1,68

Beispiel 18

Mit dem gemäß Beispiel 17 hergestellten Rhodiumkomplex wurde analog Beispiel 3 α-Acetamidozimtsäure hydriert.

Reaktionsgefäß : 0,1 l-Stahlautoklav
Einsatzmenge an Substrat : 2,05 g
Einsatzmenge an Rhodiumkomplex : 4,1 mg
Lösungsmittel : 30 ml Methanol
$H_2$-Anfangsdruck : 47 bar
Hydriertemperatur : Raumtemperatur
Hydrierzeit : 16 Stunden
Umsatz : 100 %
Optische Ausbeute : 96,0 %

**Patentansprüche** (für die Vertragsstaaten : BE, CH, DE, FR, GB, IT, LI, NL, SE)

1. Optisch aktive 3,4-Bis-(diphenylphosphino)-pyrrolidine der Formel

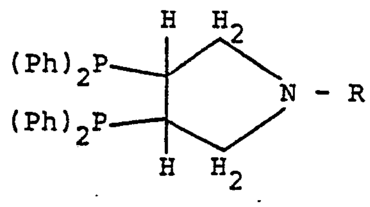

(I)

in der Ph einen Phenylrest und R Wasserstoff, einen Alkylrest, einen Arylalkylrest oder einen Acylrest bedeuten.

2. Rhodiumkomplexe der Formel

$$[Rh(en)_2A]^+ X^- \quad (II)$$

in der $(en)_2$ zwei Moleküle eines Monoolefins oder ein Molekül eines Diolefins, A ein optisch aktives 3,4-Bis-(diphenylphosphino)-pyrrolidin der Formel (I) gemäß Anspruch 1 und $X^-$ ein Tetrafluoroborat-, Hexafluorophoshat- oder Perchloratanion bedeuten.

3. Verwendung der Rhodiumkomplexe der Formel (II) gemäß Anspruch 2 als Katalysatoren für die asymmetrische Hydrierung unsubstituierter oder β-substituierter α-Acylamino-acrylsäuren.

**Patentanspruch** (für den Vertragsstaat AT)

Verwendung von Rhodiumkomplexen der Formel

$$[Rh(en)_2A]^+ X^- \quad (II),$$

in der $(en)_2$ zwei Moleküle eines Monoolefins oder ein Molekül eines Diolefins, A ein optisch aktives 3,4-Bis-(diphenylphosphino)-pyrrolidin der Formel

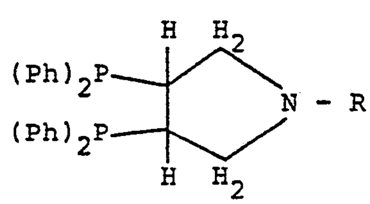

(I)

und $X^-$ ein Tetrafluoroborat-, Hexafluorophosphat- oder Perchloratanion bedeuten, wobei in der Formel (I) Ph einen Phenylrest und R Wasserstoff, einen Alkylrest, einen Arylalkylrest oder einen Acylrest darstellen, als Katalysatoren für die asymmetrische Hydrierung unsubstituierter oder β-substituierter α-Acylamino-acrylsäuren.

**Claims** (for the Contracting States : BE, CH, DE, FR, GB, IT, LI, NL, SE)

1. Optically active 3,4-bis-(diphenylphosphino)-pyrrolidines corresponding to the following formula

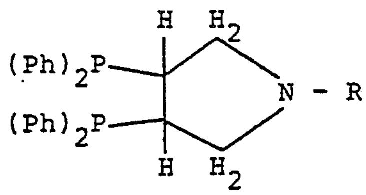

(I)

in which Ph is a phenyl radical and R is hydrogen, an alkyl radical, an arylalkyl radical or an acyl radical.

2. Rhodium complexes corresponding to the following formula

$$[Rh(en)_2A]^+ X^- \qquad (II)$$

in which $(en)_2$ represents two molecules of a monoolefin or one molecule of a diolefin, A is an optically active 3,4-bis-(diphenylphosphino)-pyrrolidine corresponding to formula (I) in claim 1 and $X^-$ is a tetrafluoroborate, hexafluorophosphate or perchlorate anion.

3. The use of the rhodium complexes corresponding to formula (II) in claim 2 as catalysts for the asymmetrical hydrogenation of unsubstituted or β-substituted α-acylaminoacrylic acids.

**Claim** (for the Contracting State AT)

The use of rhodium complexes corresponding to the following formula

$$[Rh(en)_2A]^+ X^- \qquad (II)$$

in which $(en)_2$ represents two molecules of a monoolefin or one molecule of a diolefin, A is an optically active 3,4-bis-(diphenylphosphino)-pyrrolidine corresponding to the following formula

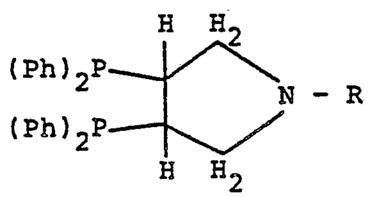

(I)

and $X^-$ is a tetrafluoroborate, hexafluorophosphate or perchlorate anion, Ph in formula (I) being a phenyl radical and R being hydrogen, an alkyl radical, an arylalkyl radical or an acyl radical, as catalysts for the asymmetrical hydrogenation of unsubstituted or β-substituted α-acylaminoacrylic acids.

**Revendications** (pour les Etats contractants : BE, CH, DE, FR, GB, IT, LI, NL, SE)

1. 3,4-bis-(diphénylphosphine)-pyrrolidines optiquement actives de formule :

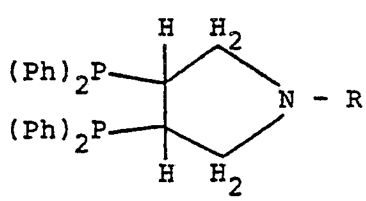

(I)

dans laquelle Ph représente un reste phényle et R représente de l'hydrogène, un reste alcoyle, un reste aralcoyle ou un reste acyle.

2. Complexes du Rhodium de formule :

$$[Rh(en)_2A]^+ X^- \qquad (II)$$

dans laquelle $(en)_2$ représente deux molécules d'une mono-oléfine ou une molécule d'une dioléfine, A représente une 3,4-bis-(diphénylphosphine)-pyrrolidine optiquement active de formule (I) selon la revendication 1 et $X^-$ est un anion tétrafluoroborate, hexafluorophosphate ou perchlorate.

3. Utilisation des complexes de Rhodium de formule (II) selon la revendication 2, en tant que catalyseurs pour l'hydrogénation asymétrique d'acides acryliques α-acylaminés non substitués ou β-substitués.

**Revendication** (pour l'Etat contractant AT)

Utilisation des complexes du rhodium de formule

$$[Rh(en)_2A]^+ X^-$$ (II)

dans laquelle $(en)_2$ représente deux molécules d'une mono-oléfine ou une molécule d'une dioléfine, A représente une 3,4-bis-(diphénylphosphine)-pyrrolidine optiquement active de formule

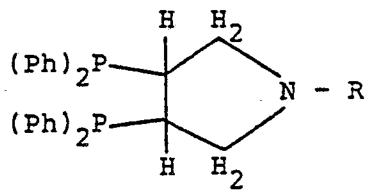

(I)

et $X^-$ représente un anion tétrafluoroborate, hexafluorophosphate ou perchlorate et où dans la formule I Ph représente un reste phényle et R représente de l'hydrogène, un reste alcoyle, un reste aralcoyle ou un reste acyle en tant que catalyseur pour l'hydrogénation asymétrique d'acides acryliques α-acylaminés non substitués ou β-substitués.